# EUROPÄISCHE PATENTSCHRIFT

(11) **EP 0 489 682 B1**
(45) Veröffentlichungstag und Bekanntmachung des Hinweises auf die Patenterteilung: **11.01.1995**
(21) Anmeldenummer: 91710044.8
(22) Anmeldetag: 18.11.1991
(51) Int. Cl.: C07C 209/68, C07B 55/00

(54) **Verfahren zur Racemisierung von optisch aktiven 1-Aryl-alkylaminen**
Process for racemization of optically active 1-aryl-alkylamines
Procédé pour la racémisation d'1-aryl-alkylamines optiquement actives

(30) Priorität: 01.12.1990 DE 4038356
(43) Veröffentlichungstag der Anmeldung: 10.06.1992
(73) Patentinhaber: BAYER AG, 51368 Leverkusen (DE)
(72) Erfinder: Jansen, Johannes R., Dr., W-4019 Monheim (DE); Littmann, Martin, Dr., W-5000 Köln 80 (DE)

(56) Entgegenhaltungen:
- DE-A- 2 441 651

## Beschreibung

Die Erfindung betrifft ein neues Verfahren zur Herstellung von racemischen Gemischen aus optisch aktiven 1-Aryl-alkylaminen der Formel (I) durch Behandeln mit Metallalkanolaten in Dimethylsulfoxid.

Optisch aktive 1-Aryl-alkylamine sind teils als handelsübliche Synthesechemikalien, teils als wertvolle organische Zwischenprodukte für Agrochemikalien, wie Herbizide oder Fungizide oder für Arzneimittel, bekannt (vgl. EP-A 300 313; EP-A 341 475). So werden die optischen Antipoden der 1-Aryl-alkylamine in isolierter Form auch als Salzbildner zur Trennung von racemischen Gemischen von optisch aktiven Säuren verwendet, da sie leicht synthetisierbar und in einfacher Weise in die Antipoden spaltbar sind. Gegenüber den in der Natur vorkommenden Alkaloiden, die früher für diesen Zweck verwendet wurden, haben sie den Vorteil, in beliebiger Menge für technische Prozesse zur Verfügung zu stehen.

Im allgemeinen werden die optisch aktiven Verbindungen der allgemeinen Formel (I) in Form von racemischen Gemischen hergestellt, die sodann in ihre optischen Antipoden gespalten werden. Nach der Abtrennung des wertvollen optischen Antipoden wird der unerwünschte Antipode wieder racemisiert und das Racemat erneut in die optischen Antipoden gespalten. Somit dient die Racemisierung indirekt zur Herstellung von erwünschten optisch aktiven Verbindungen.

Verfahren zur Racemisierung von optisch aktiven 1-Aryl-alkylaminen sind bereits bekannt. Im wesentlichen werden diese Racemisierungen in Gegenwart von metallhaltigen Katalysatoren durchgeführt, beispielsweise in Gegenwart von Naphthalin-Natrium oder Anthracen-Natrium (vgl. DE-OS 2 441 651), in Gegenwart von Natrium oder Kalium und gegebenenfalls zusätzlich von deren Hydroxiden auf Aluminiumoxid (vgl. DE-OS 2 442 845; JP-A 50 050 317 - zitiert in Chem. Abstracts 83, 96701), in Gegenwart von Natriumhydrid oder Natriumamid (vgl. DE-OS 2 348 801), oder auch in Gegenwart von Wasserstoff/Raney-Cobalt (vgl. DE-OS 2 851 039).

Die bekannten Racemisierungsverfahren sind jedoch weitgehend für den industriellen Maßstab nicht gut geeignet, sei es wegen der unbefriedigenden Ausbeute oder der ungenügenden Qualität der Produkte, sei es wegen technisch ungünstiger Katalysatoren, deren Verwendung mit hohen Sicherheitsrisiken und/oder mit Aufarbeitungsproblemen behaftet ist.

Es wurde nun ein Verfahren zur Racemisierung von optisch aktiven 1-Aryl-alkylaminen der allgemeinen Formel (I) 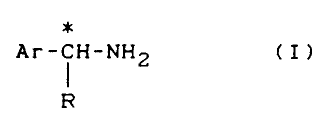
in welcher
Ar für gegebenenfalls substituiertes Aryl steht und
R für Alkyl steht,
gefunden, welches dadurch gekennzeichnet ist, daß man Verbindungen der Formel (I), welche an dem mit * markierten Kohlenstoffatom vorwiegend R- oder S-konfiguriert sind, mit Metallalkanolaten in Dimethylsulfoxid, gegebenenfalls in Gegenwart der entsprechenden Alkohole, bei Temperaturen zwischen 0°C und 200°C umsetzt.

Die optisch aktiven Verbindungen der Formel (I) können die R- und/oder S-Form in jedem Mengenverhältnis enthalten, d.h. die reinen Formen oder auch Mischungen unterschiedlicher Zusammensetzung.

Überraschenderweise können die optisch aktiven 1-Aryl-alkylamine der Formel (I) nach dem erfindungsgemäßen Verfahren einfach und schnell unter Verwendung technisch günstiger Stoffe glatt racemisiert werden. Besonders hervorzuheben ist, daß die Racemisierung auch bei halogensubstituierten Verbindungen der Formel (I) praktisch ohne Nebenreaktionen abläuft, während bei diesen Verbindungen mit den bekannten Katalysatoren Dehalogenierungen und/oder Oligomerisierungen als störende Nebenreaktionen ablaufen würden.

In der allgemeinen Formel (I) steht Ar für gegebenenfalls ein- bis fünffach, gleich oder verschieden substituiertes Aryl, vorzugsweise für Phenyl oder Naphthyl, wobei die möglichen Substituenten vorzugsweise ausgewählt sind aus der Reihe Halogen, jeweils geradkettiges oder verzweigtes Alkyl, Halogenalkyl, Alkoxy, Halogenalkoxy, Alkylthio, Alkylsulfinyl oder Alkylsulfonyl mit jeweils 1 bis 4 Kohlenstoffatomen.

Insbesondere steht Ar für Phenyl oder Naphthyl, welche jeweils gegebenenfalls durch Fluor, Chlor, Brom, Methyl, Trifluormethyl, Methoxy und/oder Trifluormethoxy einfach bis dreifach substituiert sind, ganz besonders für 4-Chlor-phenyl.
R steht in der Formel (I) für geradkettiges oder verzweigtes Alkyl, vorzugsweise mit 1 bis 6, insbesondere mit 1 bis 3 Kohlenstoffatomen, ganz besonders für Methyl.

Das erfindungsgemäße Verfahren wird insbesondere zur Racemisierung von 1-(4-Chlor-phenyl)-ethylamin durchgeführt.

Die beim erfindungsgemäßen Verfahren als Ausgangsstoffe einzusetzenden Verbindungen der Formel (I), welche an dem mit * markierten Kohlenstoffatom vorwiegend R- oder S-konfiguriert sind, sind bekannt und/oder können nach an sich bekannten Verfahren erhalten werden; man erhält sie im allgemeinen als Koppelprodukte bei der Racematspaltung von Verbindungen der Formel (I) (vgl. EP-A 341 475).

Metallalkanolate, welche beim erfindungsgemäßen Verfahren verwendet werden, sind vorzugsweise Alkalimetall-, Erdalkalimetall- oder Erdmetallalkanolate mit geradkettigen oder verzweigten Alkylgruppen, welche 1 bis 8 Kohlenstoffatome enthalten. Besonders bevorzugte Metallalkanolate sind Alkalimetallalkanolate mit 1 bis 6 Kohlenstoffatomen. Als Beispiele seien genannt:

Kalium-ethylat, -isopropylat, -sec-butylat, -tert-butylat, -sec-pentylat und -tert-pentylat (-tert-amylat).

Die Reaktionstemperaturen können bei dem erfindungsgemäßen Verfahren in einem größeren Bereich variiert werden. Im allgemeinen arbeitet man bei Temperaturen zwischen 0 °C und 200 °C, vorzugsweise bei Temperaturen zwischen 20 °C und 120 °C.

Das erfindungsgemäße Verfahren wird im allgemeinen unter Normaldruck durchgeführt. Es ist jedoch auch möglich, unter erhöhtem oder vermindertem Druck - im allgemeinen zwischen 10 hPa und 10000 hPa - zu arbeiten.

Zur Durchführung des erfindungsgemäßen Verfahrens setzt man auf 1 Mol Ausgangsverbindung der Formel (I) im allgemeinen zwischen 0,01 und 10 Mol, vorzugsweise zwischen 0,05 und 1,0 Mol, Metallalkanolat und im allgemeinen zwischen 0,5 und 20 Mol, vorzugsweise zwischen 1,0 und 10 Mol, Dimethylsulfoxid ein.

In einer bevorzugten Ausführungsform des erfindungsgemäßen Verfahrens wird die optisch aktive Ausgangsverbindung der Formel (I) bei Raumtemperatur (ca. 20° C) mit Dimethylsulfoxid vermischt, die Mischung gegebenenfalls auf eine höhere Temperatur aufgeheizt und mit dem Metallalkanolat, welches gegebenenfalls im entsprechenden Alkohol gelöst ist, versetzt.

Nach Ablauf der Racemisierungsreaktion wird nach üblichen Methoden aufgearbeitet.

Beispielsweise wird mit Salzsäure oder dem Hydrochlorid des racemischen Amins der Formel (I) das Alkanolat neutralisiert und das racemisierte Produkt der Formel (I) durch Destillation unter vermindertem Druck isoliert.

### Herstellungsbeispiele:

### Beispiel 1

1500 g (9,65 Mol) 1-(4-Chlor-phenyl)-ethylamin (Gehalt: 95 %, ¹H-NMR; S/R = 80:20, Chiral-GC) werden mit 1800 g (23,1 Mol) Dimethylsulfoxid vermischt und diese Mischung wird innerhalb von 20 Minuten auf 80° C aufgeheizt. Weitere 300 g (3,85 Mol) Dimethylsulfoxid werden als "Temperaturpuffer" in einem Tropftrichter bereitgehalten. Innerhalb einer Minute werden dann 162 g (1,45 Mol) Kalium-tert-butanolat zur Mischung gegeben, woraufhin die Innentemperatur auf ca. 90° C ansteigt. Durch Eindosieren von etwa 150 g aus dem oben erwähnten Dimethylsulfoxid-Vorrat wird ein weiteres Ansteigen der Innentemperatur verhindert. Das Reaktionsgemisch wird ca. 60 Minuten bei 85° C bis 90° C gehalten, dann auf ca. 20° C abgekühlt und mit 245 ml 6N-Salzsäure versetzt. Das Racemisierungsprodukt wird durch Destillation im Dampfstrahlvakuum isoliert.

Man erhält 1410 g (94% der Theorie) 1-(4-Chlor-phenyl)-ethylamin vom Siedepunkt 125° C/2 hPa (Gehalt: >95 %, ¹H-NMR; S/R = 50:50).

### Beispiel 2

470 g (3,02 Mol) (S)-1-(4-Chlor-phenyl)-ethylamin werden mit 611 g (7,83 Mol) Dimethylsulfoxid vermischt; dann werden 37,8 g (0,3 Mol) Kalium-tert-pentylat (Kalium-tert-amylat) dazugegeben und die Mischung wird innerhalb von 25 Minuten auf 100° C aufgeheizt. Nach 45 Minuten bei 100° C wird auf 0° C abgekühlt und 50 ml 6N-Salzsäure werden zügig zugetropft. Das Racemisierungsprodukt wird durch Destillation im Dampfstrahlvakuum isoliert.

Man erhält 432 g (92% der Theorie) (R/S)-1-(4-Chlor-phenyl)-ethylamin vom Siedepunkt 120° C/1,5 hPa.

### Beispiel 3

121 g (1,0 Mol) (R)-1-Phenyl-ethylamin werden mit 200 g (2,55 Mol) Dimethylsulfoxid vermischt; dann werden 16,8 g (0,15 Mol) Kalium-tert-butanolat dazugegeben und die Mischung wird 60 Minuten auf 90° C erhitzt. Nach Abkühlen auf 20° C wird mit 20 ml 6N-Salzsäure versetzt und das Racemisierungsprodukt wird durch Destillation unter vermindertem Druck isoliert.

Man erhält 106,5 g (88 % der Theorie) (R/S)-1-Phenylethylamin.

In den Beispielen (1) bis (3) entspricht jeweils die R-konfigurierte Verbindung der Formel (I) den rechtsdrehenden Antipoden ("(+)-Form"), die S-konfigurierte Verbindung dem linksdrehenden Antipoden ("(-)-Form").

## Patentansprüche

1. Verfahren zum Racemisieren von optisch aktiven 1-Aryl-alkylaminen, dadurch gekennzeichnet, daß man ein optisch aktives Amin der allgemeinen Formel (I) 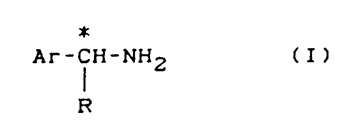 in der 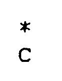 ein asymmetrisches Kohlenstoffatom darstellt,
Ar für gegebenenfalls substituiertes Aryl und
R für Alkyl stehen, mit Metallalkanolaten in Dimethylsulfoxid, gegebenenfalls in Gegenwart von entsprechenden Alkoholen behandelt.

2. Verfahren nach Anspruch 1, dadurch gekennzeichnet, daß das optisch aktive Amin in reiner Form oder in Mischung unterschiedlicher Zusammensetzung vorliegen kann.

3. Verfahren nach Anspruch 1, dadurch gekennzeichnet, daß die Metallalkanolate Alkali-, Erdalkali- oder Erdmetallalkanolate sind.

4. Verfahren nach Anspruch 1, dadurch gekennzeichnet, daß man bei Temperaturen zwischen 0°C und 200°C arbeitet.

5. Verfahren nach Anspruch 1, dadurch gekennzeichnet, daß in der Formel (I)
Ar für gegebenenfalls einfach bis fünffach, gleich oder verschieden durch Halogen, jeweils geradkettiges oder verzweigtes Alkyl, Halogenalkyl, Alkoxy, Halogenalkoxy, Alkylthio, Alkylsulfinyl oder Alkylsulfonyl mit jeweils 1 bis 4 Kohlenstoffatomen substituiertes Phenyl oder Naphthyl steht und
R für geradkettiges oder verzweigtes Alkyl mit 1 bis 6 Kohlenstoffatomen steht.

6. Verfahren nach Anspruch 5, dadurch gekennzeichnet, daß in der Formel (I)
Ar für einfach bis dreifach, gleich oder verschieden durch Fluor, Chlor, Brom, Methyl, Trifluormethyl, Methoxy oder Trifluormethoxy substituiertes Phenyl oder Naphthyl und
R für geradkettiges oder verzweigtes Alkyl mit 1 bis 3 Kohlenstoffatomen stehen.

## Claims

1. Process for racemization of optically active 1-aryl-alkylamines, characterized in that an optically active amine of the general formula (I) 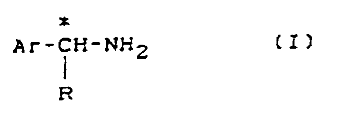 in which 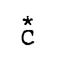 represents an asymmetric carbon atom,
Ar represents optionally substituted aryl and
R represents alkyl, is treated with metal alkanolates in dimethyl sulphoxide, if appropriate in the presence of corresponding alcohols.

2. Process according to Claim 1, characterized in that the optically active amine can be present in the pure form or in a mixture of varying composition.

3. Process according to Claim 1, characterized in that the metal alkanolates are alkali metal, alkaline earth metal or earth metal alkanolates.

4. Process according to Claim 1, characterized in that it is carried out at temperatures between 0°C and 200°C.

5. Process according to Claim 1, characterized in that, in formula (I),
Ar represents phenyl or naphthyl which are optionally monosubstituted to pentasubstituted in an identical or different manner by halogen or in each case straight-chain or branched alkyl, halogenoalkyl, alkoxy, halogenoalkoxy, alkylthio, alkylsulphinyl or alkylsulphonyl having in each case 1 to 4 carbon atoms and
R represents straight-chain or branched alkyl having 1 to 6 carbon atoms.

6. Process according to Claim 5, characterized in that, in formula (I),
Ar represents phenyl or naphthyl which are monosubstituted to trisubstituted in an identical or different manner by fluorine, chlorine, bromine, methyl, trifluoromethyl, methoxy or trifluoromethoxy and
R represents straight-chain or branched alkyl having 1 to 3 carbon atoms.

## Revendications

1. Procédé de racémisation de 1-arylalkylamines optiquement actives, caractérisé en ce qu'on traite une amine optiquement active de formule générale (I) : 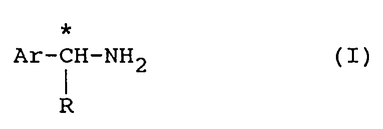 dans laquelle 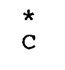 représente un atome asymétrique de carbone,
Ar désigne un groupe aryle éventuellement substitué et
R est un groupe alkyle,
avec des alcanolates métalliques dans du diméthylsulfoxyde, le cas échéant en présence d'alcools correspondants.

2. Procédé suivant la revendication 1, caractérisé en ce que l'amine optiquement active peut se présenter sous la forme pure ou sous la forme d'un mélange de composition variée.

3. Procédé suivant la revendication 1, caractérisé en ce que les alcanolates métalliques sont des alcanolates de métaux alcalins, de métaux alcalino-terreux ou de terres rares.

4. Procédé suivant la revendication 1, caractérisé en ce qu'il est mis en oeuvre à des températures comprises entre 0°C et 200°C.

5. Procédé suivant la revendication 1, caractérisé en ce que, dans la formule (I) :
Ar est un groupe phényle ou naphtyle portant le cas échéant 1 à 5 substituants, égaux ou différents, halogéno, alkyle, halogénalkyle, alkoxy, halogénalkoxy, alkylthio, alkylsulfinyle ou alkylsulfonyle linéaires ou ramifiés ayant chacun 1 à 4 atomes de carbone et
R est un groupe alkyle linéaire ou ramifié ayant 1 à 6 atomes de carbone.

6. Procédé suivant la revendication 5, caractérisé en ce que dans la formule (I) :
Ar représente un groupe phényle ou naphtyle portant 1 à 3 substituants, égaux ou différents, fluoro, chloro, bromo, méthyle, trifluorométhyle, méthoxy ou trifluorométhoxy et
R est un groupe alkyle linéaire ou ramifié ayant 1 à 3 atomes de carbone.
